# EUROPEAN PATENT SPECIFICATION

(11) **EP 3 977 075 B1**
(45) Date of publication and mention of the grant of the patent: **11.10.2023**
(21) Application number: 20747093.1
(22) Date of filing: 25.05.2020
(51) Int. Cl.: G01J 3/44, G01N 21/65, G01N 33/48, G01N 33/564

(54) **CELIAC DISEASE DIAGNOSIS METHOD**
VERFAHREN ZUR DIAGNOSE VON ZÖLIAKIE
MÉTHODE DE DIAGNOSTIC DE MALADIE COELIAQUE

(30) Priority: 24.05.2019 IT 201900007214
(43) Date of publication of application: 06.04.2022
(73) Proprietor: IRCCS Centro Neurolesi "Bonino-Pulejo", 98124 Messina (IT)
(72) Inventor: ACRI, Giuseppe, 87045 Dipignano (IT); BRAMANTI, Placido, 98124 Messina (IT); VERMIGLIO, Giuseppe, 98158 Messina (IT); BRAMANTI, Alessia, 98121 Messina (IT); TESTAGROSSA, Barbara, 98168 Messina (IT); MARINO, Silvia, 98121 Messina (IT); COSTA, Stefano, 98125 Messina (IT); CIURLEO, Rosella, 89050 Feroleto della Chiesa (IT)
(74) Representative: Celona, Antonio
(86) International application number: PCT/IB2020/054939
(87) International publication number: WO 2020/240399

(56) References cited:
- CN-A- 107 192 704
- CN-A- 109 580 584
- US-A1- 2010 114 514
- STEFANO FORNASARO ET AL: "Potential use of MCR-ALS for the identification of coeliac-related biochemical changes in hyperspectral Raman maps from pediatric intestinal biopsies", INTEGRATIVE BIOLOGY, vol. 10, no. 6, 1 January 2018 (2018-01-01), pages 356-363, XP055661701, Cambridge ISSN: 1757-9694, DOI: 10.1039/C8IB00028J
- MANOHARAN R ET AL: "Histochemical analysis of biological tissues using Raman spectroscopy", SPECTROCHIMICA ACTA. PART A: MOLECULAR AND BIOMOLECULAR SPECTROSCOPY, ELSEVIER, AMSTERDAM, NL, vol. 52, no. 2, 1 February 1996 (1996-02-01), pages 215-249, XP027532228, ISSN: 1386-1425 [retrieved on 1996-02-01]
- R VALDÉS ET AL: "Pilot research on the evaluation and detection of head and neck squamous cell carcinoma by Raman spectroscopy", JOURNAL OF RAMAN SPECTROSCOPY, vol. 45, no. 7, 27 May 2014 (2014-05-27), pages 550-557, XP055585327, GB ISSN: 0377-0486, DOI: 10.1002/jrs.4498
- MAZIAK ET AL: "Fourier-transform infrared spectroscopic study of characteristic molecular structure in cancer cells of esophagus: An exploratory study", CANCER DETECTION AND PREVENTION, ELSEVIER SCIENCE, NL, vol. 31, no. 3, 1 January 2007 (2007-01-01), pages 244-253, XP022201834, ISSN: 0361-090X, DOI: 10.1016/J.CDP.2007.03.003
- MILLER SARA ET AL: "Su2061 - A Multi-Spectroscopic Approach to Disease Diagnosis: A Proof-Of-Principle Study on Ex Vivo Detection of Coeliac Disease", GASTROENTEROLOGY : OFFICIAL PUBLICATION OF THE AMERICAN GASTROENTEROLOGICAL ASSOCIATION, vol. 156, no. 6, 1 May 2019 (2019-05-01), - 1 May 2019 (2019-05-01), XP085678709, ISSN: 0016-5085, DOI: 10.1016/S0016-5085(19)38683-4
- FENG SHANGYUAN ET AL: "A noninvasive cancer detection strategy based on gold nanoparticle surface-enhanced raman spectroscopy of urinary modified nucleosides isolated by affinity chromatography", BIOSENSORS AND BIOELECTRONICS, ELSEVIER SCIENCE LTD. UK, AMSTERDAM, NL, vol. 91, 5 January 2017 (2017-01-05), pages 616-622, XP029920719, ISSN: 0956-5663, DOI: 10.1016/J.BIOS.2017.01.006
- PENCE I J ET AL: "Endoscopy-coupled Raman spectroscopy for in vivo discrimination of inflammatory bowel disease", PROGRESS IN BIOMEDICAL OPTICS AND IMAGING, SPIE - INTERNATIONAL SOCIETY FOR OPTICAL ENGINEERING, BELLINGHAM, WA, US, vol. 8939, 4 March 2014 (2014-03-04), pages 89390R-89390R, XP060033690, ISSN: 1605-7422, DOI: 10.1117/12.2042795 ISBN: 978-1-5106-0027-0
- SHANGYUAN FENG ET AL: "Gastric cancer detection based on blood plasma surface-enhanced Raman spectroscopy excited by polarized laser light", BIOSENSORS AND BIOELECTRONICS, ELSEVIER SCIENCE LTD. UK, AMSTERDAM, NL, vol. 26, no. 7, 9 December 2010 (2010-12-09), pages 3167-3174, XP028148394, ISSN: 0956-5663, DOI: 10.1016/J.BIOS.2010.12.020 [retrieved on 2010-12-17]

## Description

### Field of the invention

The present invention relates to a method for diagnosing celiac disease (CD).

### Background art

Celiac disease (CD) is an immune-mediated systemic disease induced in genetically predisposed subjects by the ingestion of proteins rich in proline and glutamine residues contained in wheat, rye, and barley.

According to the official classification of CD (Marsh-Obehuber), the intestinal damage progresses through various stages up to the final stage of intestinal villous atrophy (Marsh 3), while the initial stage is represented by intraepithelial lymphocyte infiltration (Marsh 1) and crypt hypertrophy (Marsh 2). CD is defined by a very broad clinical spectrum and is associated with an increased risk of morbidity and mortality. Patients may be absolutely asymptomatic or show even severe clinical symptoms. The spectrum of CD may be divided into 4 main categories.

The classic form, mainly diagnosed during early childhood, is characterized by duodenal atrophy associated with typical intestinal malabsorption symptoms, such as globular abdomen, weight loss, diarrhea, stature-weight retardation, hypoprotidemia.

In the non-classical form, which is continuously increasing, the classic intestinal symptoms are not present, generic intestinal symptoms are often present and extraintestinal signs and symptoms may be present, such as dermatitis, iron-deficiency anemia, hepatitis, cholangitis, hypertransaminasemia, coagulopathy, delayed puberty, osteopenia, arthralgias, aphthous stomatitis, dental enamel defects, alopecia, edema, infertility, depression, cerebellar ataxia.

The potential form is defined by the presence of positive serology, compatible HLA (DQ2 or DQ8) in the absence of duodenal atrophy. Intestinal and extra-intestinal signs and symptoms may or may not be present.

In the silent form, patients are apparently asymptomatic.

CD diagnosis is based on the presence of characteristic lesions in the duodenal biopsy.

Multiple biopsy fragments are required to have a sufficient diagnostic accuracy, and the orientation of the fragments is fundamental for a correct diagnosis. The aforementioned Marsh classification, modified by Oberhuber, is currently used for the histological diagnosis of CD. Partial (Marsh 3A) to total (Marsh 3C) villous atrophy is diagnostic for CD, but only in the presence of a positive serology since similar lesions are found in other pathologies. Marsh stage II, represented by crypt hypertrophy and intraepithelial lymphocyte infiltration, is considered sufficient to diagnose CD in children, in the presence of positive serology, according to what is established by the new ESPGHAN guidelines. Intraepithelial lymphocyte infiltration (IEL), more specifically the presence of more than 25 CD3+ lymphocytes per 100 enterocytes, is less specific for CD and recently other causes of duodenal lymphocytosis have been described and should be considered in the differential diagnosis.

The serological markers usually evaluated for the diagnosis of celiac disease are anti-transglutaminase antibodies (tTG-As) and anti-endomysial antibodies (EMAs). The sensitivity of the EMAs (IgA class) varies in the various studies from 86% to 100% (average 95%) while the specificity stands at values ranging from 90% to 100% (average 99%). For tTG-As (IgA class) sensitivity varies from 61% to 100% (average 85%) and specificity from 86% to 100% (average 95%). Overall, therefore, the combination of EMAs and tTG-As shows good diagnostic accuracy. A new class of antibodies, against deamidated gliadin peptides (DGP-Abs), have shown to be equally accurate compared to EMAs and tTG-As, if not superior in the follow-up for assessing compliance with a gluten-free diet.

However, disadvantageously, in most cases the current background art does not allow a reliable diagnosis of celiac disease to be made in a non-invasive manner, in particular without performing at least one duodenal biopsy.

Although since 2012 the CD diagnosis guidelines of the European Society for Paediatric Gastroenterology Hepatology and Nutrition (ESPGHAN) have introduced the possibility of avoiding duodenal biopsy in pediatric patients, it is also true that this type of diagnostic path is only possible for a minority percentage of patients. In fact, for diagnosing CD without biopsy it is necessary that patients have, at the same time, one of the classic symptoms associated with CD (intestinal malabsorption) and a positivity of the specific serology (anti-transglutaminase antibodies) with a level equal to at least 10 times the normal limit provided for the test. In these patients, confirmation with a further sampling is then required, to determine the anti-endomysial antibodies and HLA (histocompatibility haplotype). In case of positive anti-endomysial antibodies and compatible HLA, the diagnosis may be formalized. Disadvantageously, given the increase in the incidence of CD, considering that all the guidelines for the adult involve biopsy diagnosis and that, both in adults and in children, asymptomatic cases or cases with atypical symptomatology are increasing, it can be deduced that in the near future the number of patients who will have to undergo an endoscopic examination for the biopsy diagnosis of CD will tend to increase rather than decrease.

The article "Potential use of MCR-ALS for the identification of coeliac-related biochemical changes in hyperspectral Raman maps from paediatric intestinal biopsies", INTEGRATIVE BIOLOGY, 20180101, Stefano Fornasaro, Vol. 10, Nr. 6, pages 356-363 discloses a diagnosis method for classifying coeliac disease patients (CDs) and healthy control patients (HCs). The method uses Raman hyperspectral imaging of a human sample such as tissue from colon, intestine, small-bowel mucosal biopsies or duodenum whereby three spectral regions: 1060 to 1124 cm-1, 1240 to 1300 cm-1 and 1654 cm-1 are selected. Confirmation that the sample belongs to a celiac patient is based on the Raman difference spectrum between the measured suspected CD patient spectrum and a healthy control (HC) patient spectrum at the three spectral regions.

### Summary of the invention

It is an object of the present invention to provide an alternative method for diagnosing celiac disease (CD), which is based on Raman spectrophotometric analysis, operating directly on human serum in a non-invasive and non-destructive manner.

The present invention achieves the aforesaid objects by providing a method as set out in the appended claims.

Deconvolution may be used to recognize peaks within a band, when the latter is noisy, as well as to clean up the tails of the band affected by noise and overlapping. Therefore, performing such a mathematical operation allows to clean the spectrum from the observed noise and to identify the possible presence of other peaks, not visible on the original spectrum. As a result of such a cleaning, the sum of the areas of the individual Gaussians related to the second band and the sum of the areas of the individual Gaussians related to the third band, obtained with the deconvolution performed in the regions of interest, are different and more representative, with respect to the area of the band considered and referable to the original spectrum.

With the aim of identifying a procedural path which exclusively takes into account a physical investigation technique, such as the Raman spectroscopy, a methodology has been developed based on the analysis of the serum of patients by means of the Raman spectrometer.

The new methodology of the present invention has several advantages:
- extremely low cost, with respect to the currently used serological techniques, which are based on the ELISA technique, which requires the use of special diagnostic kits;
- simplicity of use, experimental repeatability, and speed of execution;
- the technique is not invasive;
- the technique is not destructive; in fact, the sample in question can be stored and analyzed several times over time, since the only limit of the analysis is given by the degradation of the sample itself.
It is one of the objectives to add a highly performing diagnostic test to the battery of serological tests with the aim of totally avoiding invasive tests. The methodology suggested for the diagnosis of Celiac Disease (CD) is based solely on the analysis of human serum by means of the use of Raman spectroscopy and the consequent analysis of the spectrum by calculating the ratio between well-defined areas of the region. Starting from a serum sample, which contains information on the whole human body, and not information attributable to an individual pathology, it was possible to identify three regions, whose areas A₁, A₂ and A₃, taken individually, are not capable of discriminating the patient affected by CD with respect to the healthy one, but, by means of the A₃/A₁ and A₂/A₁ ratios, it is possible to diagnose CD in patients, with at least a 97% reliability proven by statistical analysis.

Further features and advantages of the invention will become apparent in light of the detailed description of some exemplary but not exclusive embodiments.

The dependent claims describe particular embodiments of the invention.

### Brief description of the drawings

In the description of the invention, reference is made to the accompanying drawings, which are provided by way of non-limiting example, in which:
Figure 1 shows Raman spectra of a serum sample from a healthy subject (a) and an ill subject (b), respectively;
Figure 2 shows a first band of the Raman spectra of Figure 1;
Figure 3 shows, together, a second band and a third band of the Raman spectra of Figure 1;
Figure 4a shows a deconvolution involving the second band of the Raman spectrum of the healthy subject;
Figure 4b shows a deconvolution involving the second band of the Raman spectrum of the ill subject;
Figure 5a shows a deconvolution involving the third band of the Raman spectrum of the healthy subject;
Figure 5b shows a deconvolution involving the third band of the Raman spectrum of the ill subject;
Figure 6a shows a ROC curve related to a first ratio of areas to measure the accuracy of the diagnostic test;
Figure 6b shows a ROC curve related to a second ratio of areas to measure the accuracy of the diagnostic test.

### Detailed description of embodiments of the invention

The method of the invention for diagnosing celiac disease is as set out in the appended claims.

Preferably, between step c) and d) a cleaning of the second band and third band of the spectrum from any possible observed noise is provided.

If this cleaning is performed, the sum A₂ of the areas of the individual Gaussians related to the second band and the sum A₃ of the areas of the individual Gaussians related to the third band, obtained by means of the deconvolution, are different, respectively, from the area A₂' of the second band and the area A₃' of the third band of the original Raman spectrum, i.e., of the Raman spectrum of step a).

Phenylalanine was selected as the first indicator; phospholipids were selected as the second indicator; and amide-I was selected as the third indicator.

The first band is within a first sub-range of wavenumbers, preferably between about 1015 cm⁻¹ and 990 cm⁻¹; the second band is within a second sub-range of wavenumbers, preferably between about 1500 cm⁻¹ and 1400 cm⁻¹; and the third band is within a third sub-range of wavenumbers, preferably between about 1750 cm⁻¹ and 1550 cm⁻¹.

In particular, the third band, for example centered near the 1650 cm⁻¹, was assigned to the vibration modes of the amide-I, which mainly involves C=O stretching and, to a lesser extent, *C-N* stretching, *C_{α}-C-N* bending vibrations, and *N-H* bending vibrations in plane of the peptide groups.

The second band, for example centered near the 1450 cm⁻¹, was assigned to the phospholipid vibration modes which involve the bending vibrations of the groups *CH₂* and *CH₃*; while the first band, for example, centered near the 1005 cm⁻¹, was assigned to the phenylalanine vibration mode, which involves the breathing mode of the phenylalanine aromatic ring.

Preferably, the first threshold value and the second threshold value can be defined by performing the following steps:
- providing a Raman spectrum of a blood serum sample for each patient of a first known group of celiac patients and for each patient of a second known group of non-celiac patients;
- for each patient, both of the first group and of the second group, selecting from the respective Raman spectrum the first band characteristic of the first indicator of the presence of celiac disease, the second band characteristic of the second indicator of the presence of celiac disease, and the third band characteristic of the third indicator of the presence of celiac disease;
- performing for each Raman spectrum a deconvolution of at least the second band and the third band, obtaining a respective plurality of Gaussians; calculating the first sum A₂ of the areas of the individual Gaussians related to the second band, and the second sum A₃ of the areas of the individual Gaussians related to the third band; and calculating the first ratio A₂/A₁ and the second ratio A₃/A₁, wherein A₁ is the area under the first band of the Raman spectrum;
- performing an analysis of a first ROC curve, obtained by considering the first ratios A₂/A₁ as a database, and determining, in a known manner, the first optimal threshold value using the Youden index, where the aforesaid Youden index is obtained by means of the homonymous function, which depends on sensitivity and specificity, which in turn depend on the considered cut-off value. The cut-off value at which the Youden index is maximum therefore represents the optimal cut-off value;
- performing an analysis of a second ROC curve, obtained by considering the second ratios A₃/A₁ as the database, and determining, similarly to what was described above, the second optimal threshold value using the Youden index.

As is known, most diagnostic tests produce a quantitative result. To discriminate between healthy and ill people it is necessary to have a threshold or cut-off value. In an ideal situation, healthy and ill people return different test values and the cut-off value is immediately determined. In real situations, there is always some overlap in the distribution of healthy and ill people. Sensitivity and specificity are inversely related in relation to the selection of the cut-off. The adoption of a threshold which offers high sensitivity leads to a loss of specificity and vice versa.

In the proposed method, although the distribution of healthy and ill people is distinct enough, to obtain the threshold values which minimize the probability of finding false positives and false negatives, it is preferable to construct two ROC (Receiver Operating Characteristic) curves in a known manner, with sensitivity on the ordinates and (1-specificity) on the abscissas, considering the first ratios A₂/A₁ and the second ratios A₃/A₁, respectively, as the database, therefore obtaining the first optimal threshold value and the second optimal threshold value.

It was found that the second threshold value, related to the ratios A₃/A₁, is greater than the first threshold value, related to the ratios A₂/A₁.

The information obtained from a Raman scattering experiment is graphically depicted as a diagram (Raman spectrum) where the abscissas report the Raman shifts corresponding to the energy jumps between the fundamental vibrational levels and expressed in cm⁻¹ (wavenumber, keeping in mind the direct proportionality between the energy and the inverse of the wavelength of an electromagnetic radiation). Raman intensities proportional to the number of Stokes photons collected by the instrument detector are shown on the ordinates. The range of energies reported in a normal Raman spectrum can extend from a few tens of cm⁻¹ up to about 3500 cm⁻¹, a region in which almost all the fundamental molecular vibrations fall.

In particular, the Raman spectrum of the blood serum sample can be acquired in a range of wavenumbers between 3500 cm⁻¹ and 300 cm⁻¹, preferably between 3300 cm⁻¹ and 400 cm⁻¹, even more preferably between 2500 cm⁻¹ and 500 cm⁻¹. Raman spectroscopy can be performed by means of any Raman spectrometer having a laser source, such as, for example, a diode laser source, with a wavelength preferably, but not necessarily, equal to 780 nm.

The spectrometer is connected to a detector, preferably a charge-coupled device (CCD), possibly integrated with a monochromator.

The Raman spectrum can, for example, be acquired by performing a number of scans of the serum sample between 25 and 35, with an exposure time between 30 and 90 seconds for each scan.

Preferably the parameter selected to perform the deconvolution, in addition to the respective band of the Raman spectrum, is the full width at half maximum (FWHM) of the respective band, expressed in wavenumbers.

An experimental work is described below, which allowed developing the method of the invention.

Serum samples from healthy subjects, not affected by celiac disease, and from celiac subjects, with diagnosis proven by duodenal biopsy, have been analyzed. In total, 263 patients have been analyzed, including 21 adults and 242 children (3-16 years).

The serum samples were analyzed, 3-4 hours after the serum sampling, by means of a DXR Smart Raman spectrometer, which has a diode laser with a wavelength of 780 nm as source.

The spectra were acquired in a range of wavenumbers between 3300 cm⁻¹ and 400 cm⁻¹, performing 32 scans of the serum sample, with an exposure time of 60 seconds for each scan. The laser was used at the maximum power provided by the system (24 mW) at the output of a 50 µm pinhole opening.

The detector connected to the DXR Smart Raman is a charge-coupled device (CCD) which, in addition to having very high sensitivity, allows to simultaneously study a broad spectral band, instead of a single wavelength at a time. In particular, the use of a CCD integrated with a monochromator allows to obtain, in one go, the entire Raman spectrum with advantages in terms of experimental simplicity and speed of execution of the experiment.

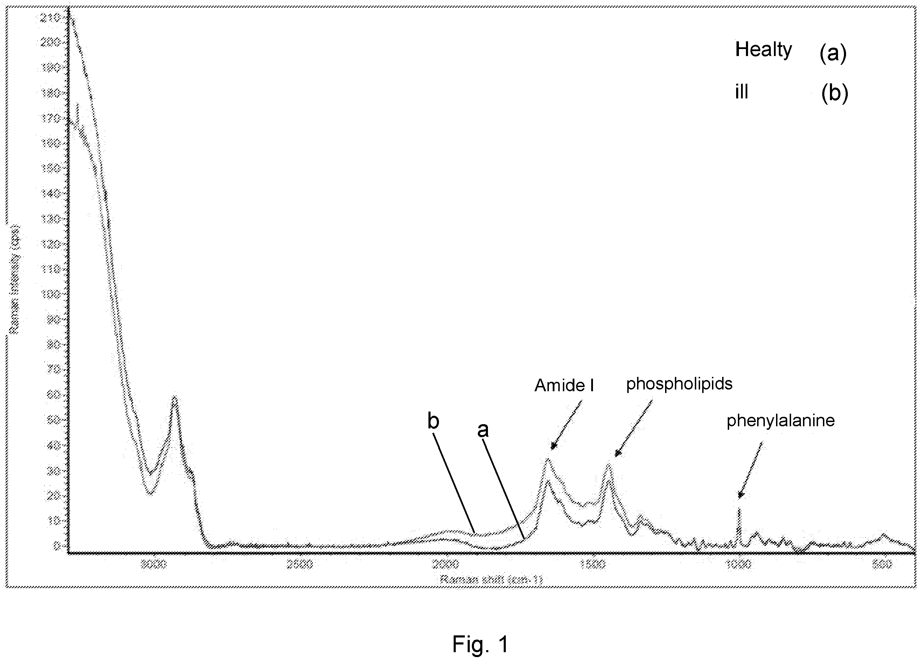

As an example, the spectra of a healthy subject and a celiac subject are shown in the Figures. The spectra obtained have a typical trend depicted in Figure 1, where the Raman spectra (3300 cm⁻¹ - 400 cm⁻¹) of a healthy subject (a) and of a subject with celiac disease (b) are shown. Figure 1 also highlights the bands of interest for the present study. The following are indicators of the presence of celiac disease:
- phenylalanine, preferably the breathing vibration mode of the phenylalanine aromatic ring, corresponding to the band between 1015 cm⁻¹ and 990 cm⁻¹;
- phospholipids, preferably the phospholipid vibration modes, corresponding to the band between 1500 cm⁻¹ and 1400 cm⁻¹;
- amide-I, preferably the amide-I vibration modes, corresponding to the band between 1750 cm⁻¹ and 1550 cm⁻¹.

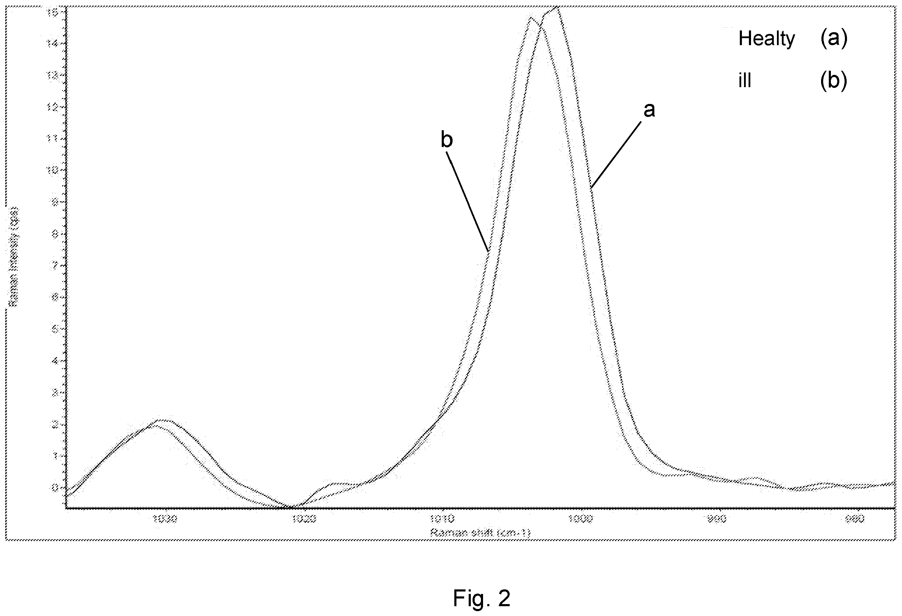

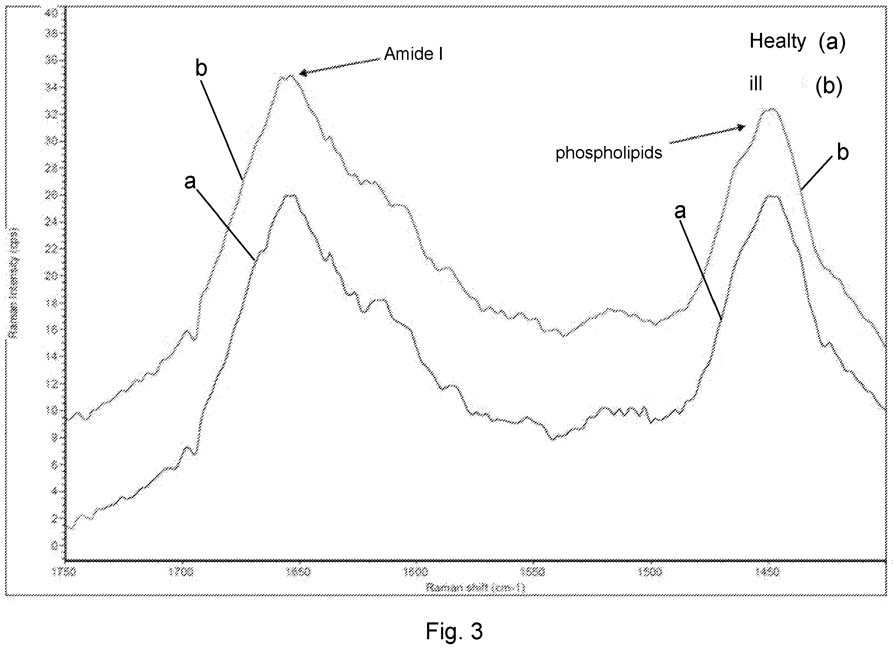

Figures 2 and 3 better highlight, in detail, the regions of interest, always with reference to the comparison between healthy subject (a) and ill subject (b).

It has been found that, while the band relating to the breathing vibration mode, or simply breathing mode, of the phenylalanine aromatic ring has a substantially indistinguishable peak between healthy and ill subjects, the other two bands have a fairly variable trend. For example, for some healthy subjects, the Raman intensity is lower than that of ill subjects, while for other healthy subjects the Raman intensity is greater than that of ill subjects.

It was therefore decided to develop a procedure for the identification of subjects suffering from celiac disease based on the relationship between the bands of interest. The band related to the breathing vibration mode of the phenylalanine aromatic ring has been used to normalize the spectra. Such a region, in fact, is not sensitive to the changes in the conformation of proteins, and is therefore used to normalize the Raman spectra of proteins.

In particular, the following two bands can be deconvolved:
- the band between 1500 cm⁻¹ and 1400 cm⁻¹;
- and the band between 1750 cm⁻¹ and 1550 cm⁻¹;

Deconvolution is a well-known mathematical operation which allows resolving overlapping or very close bands. The parameters selected to perform the above operation are:
- the shape of the respective band;
- the full width at half maximum of the band (FWHM), expressed in wavenumbers. A Gaussian fit with high sensitivity was chosen to be performed and, for the 1500 cm⁻¹-1400 cm⁻¹ and 1750 cm⁻¹-1550 cm⁻¹ bands, an FWHM equal to 20.

The spectrum, in such regions, was therefore deconvolved and, once the noise possibly observed was cleaned, the areas of all the Gaussians present therein were added (these sums of areas being called A₂ and A₃).

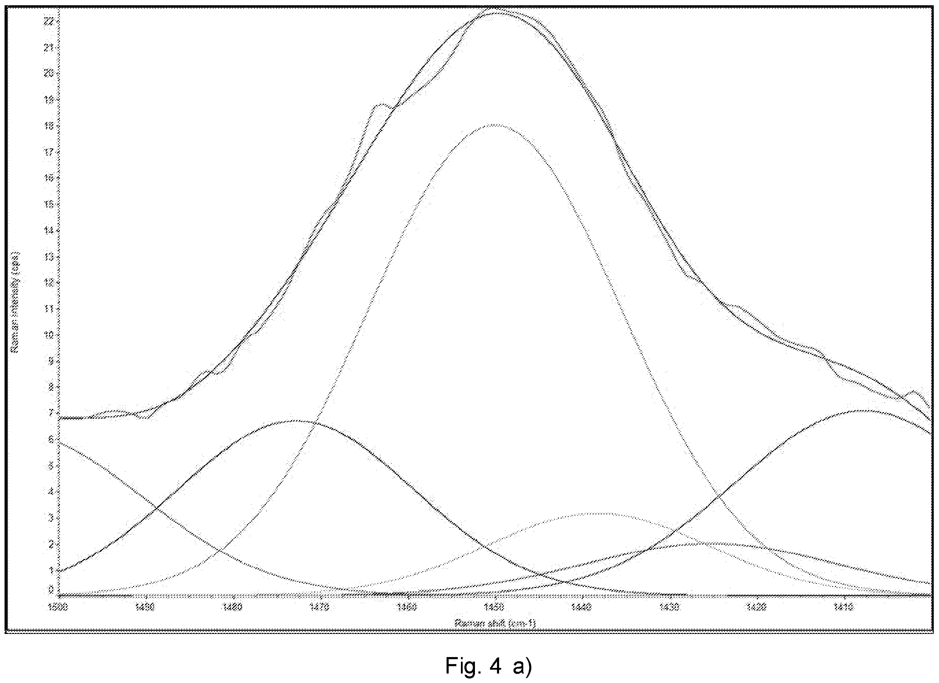

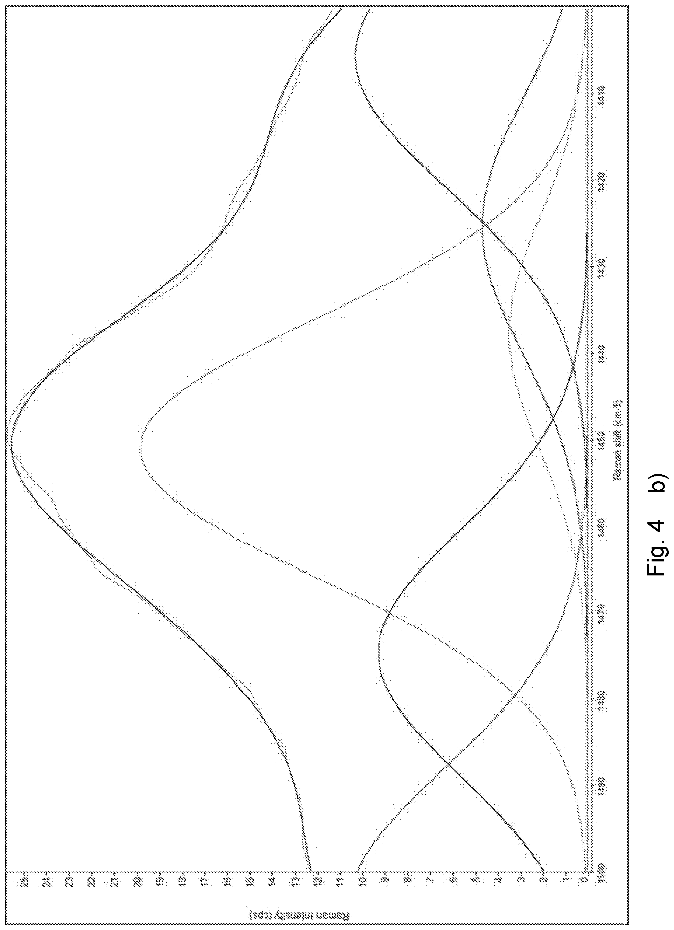

Figure 4 shows, by way of example, the deconvolved spectra, by means of Gaussian functions, in the range between 1500 cm⁻¹ and 1400 cm⁻¹ of a healthy subject (a) and of an ill subject (b), respectively.

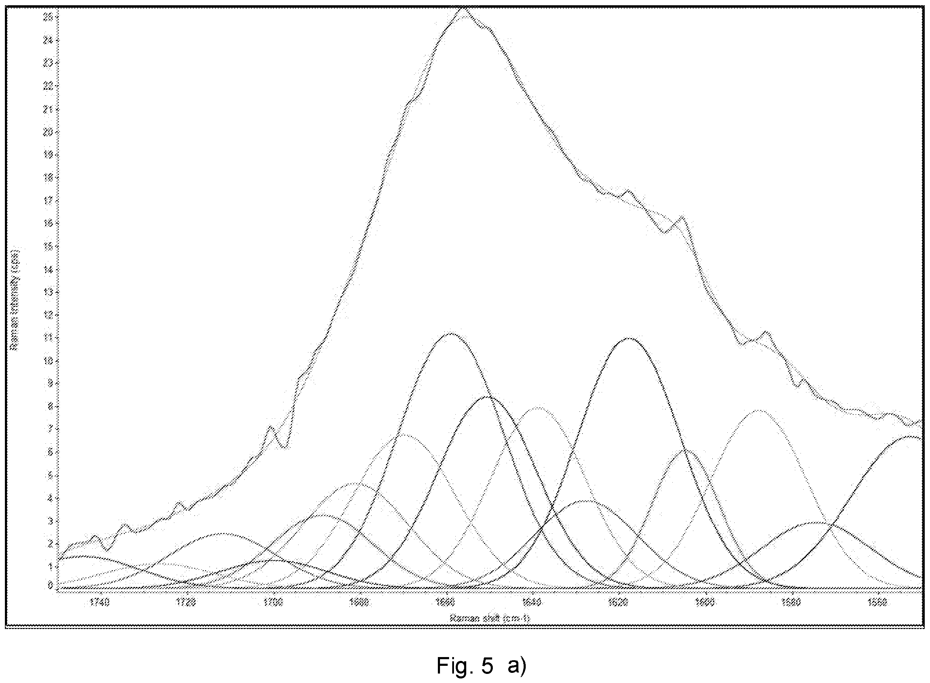

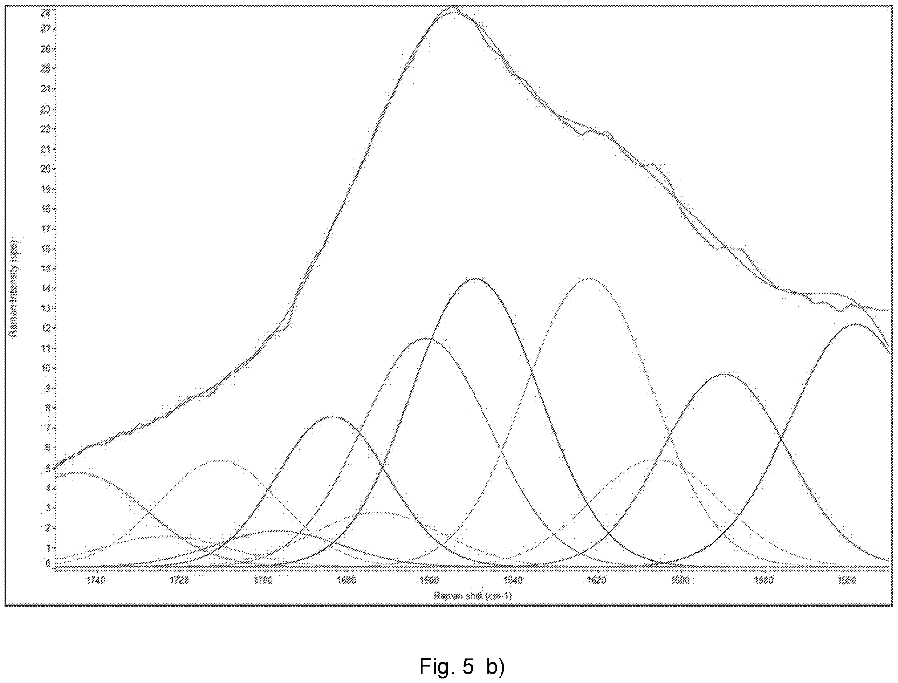

Figure 5 shows the deconvolved spectra, by means of Gaussian functions, in the range between 1750 cm⁻¹ and 1550 cm⁻¹ of a healthy subject (a) and of an ill subject, respectively.

Such sums of areas A₂ and A₃ were therefore compared to area A₁, under the peak related to the breathing vibration mode of the phenylalanine aromatic ring and calculated in the range between 1015 cm⁻¹ and 990 cm⁻¹.

Such A₂/A₁ and A₃/A₁ ratios return significantly different values between celiac and non-celiac patients.

Table 1 shows the comparison between a healthy subject and a subject affected by celiac disease.

Such a comparison showed that the A₂/A₁ and A₃/A₁ ratios are greater in the case of the celiac subject. By repeating the procedure on 261 other subjects, ill and healthy, the same behavior was found.

**Table 1**

| **Areas Ratio** | **healthy patient** | **ill patient** |
|---|---|---|
| A₂/A₁ | 13.38 | 31.85 |
| A₃/A₁ | 24.61 | 57.38 |

The analysis of the ROC (Receiver Operating Characteristic) curve was therefore used to measure the accuracy of the diagnostic test along the whole range of possible values.

The area under the ROC curve, also called AUC (Area Under the Curve), defines the diagnostic accuracy of the test and a diagnostic test with AUC having a value ≥ 80% is considered accurate.

For the interpretation of the values of the area under the ROC curve, the classification suggested by Swets is used, i.e.:

| | |
|---|---|
| AUC=0.5 | non-informative test |
| 0.5<AUC≤0.7 | inaccurate test |
| 0.7<AUC≤0.9 | moderately accurate test |
| 0.9<AUC<1.0 | highly accurate test |
| AUC=1.0 | perfect test. |

In order to identify the optimal threshold value for the A₂/A₁ and A₃/A₁ ratios (the so-called best cut-off value), i.e., the value of the test which maximizes the difference between the true positives (i.e., the proportion of individuals who have a value of the test altered, among all those really affected by the disease) and the false positives (i.e. the proportion of individuals who, despite having a value of the test altered, are not affected by the disease), the Youden index was used.

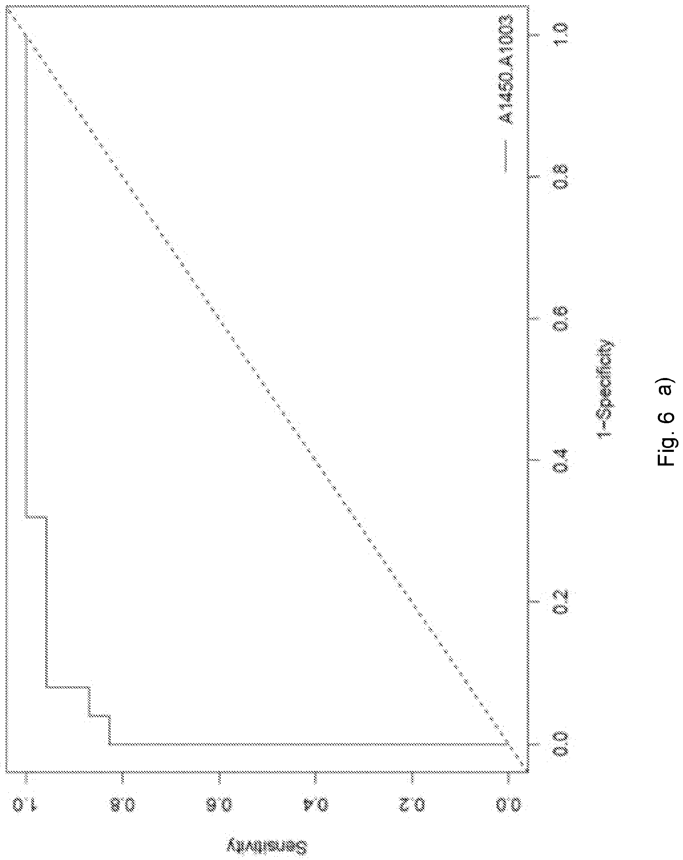

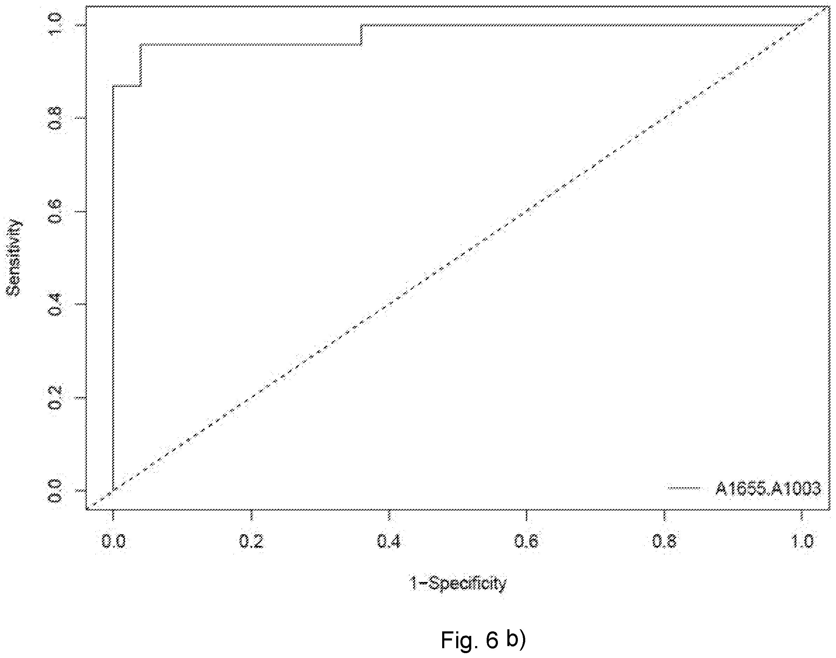

Figures 6a and 6b show the ROC curves related to the A₂/A₁ and A₃/A₁ ratios, respectively, while in Table 2 the cut-off values obtained from the analysis of these curves are indicated.

**Table 2**

| **Areas Ratio** | **Cut-off** |
|---|---|
| A₂/A₁ | 21.97 |
| A₃/A₁ | 40.14 |

By analyzing the serum of the 263 patients in the manner described above, an A₂/A₁ ratio lower than 21.97 and an A₃/A₁ ratio lower than 40.14 can be used as a discriminator for healthy subjects.

Advantageously, both ratios are indicative of the presence of celiac disease, as shown by the statistical tests reported below.

In fact, such threshold values were chosen as reference values and, on this basis, the diagnostic efficiency test was performed, calculating sensitivity (i.e., the ability to correctly identify the ill subjects) and diagnostic specificity (i.e., the ability to correctly identify the healthy subjects) of the new method.

The results obtained were reported in Tables 3 and 4, related to the A₂/A₁ and A₃/A₁ ratios, respectively.

The same tables also indicate the lower limit and the upper limit of sensitivity and specificity, calculated taking into account a 95% confidence interval.

**Table 3: Sensitivity and Specificity related to the ratio A₂/A₁**

| **A₂/A₁** | **Estimate (%)** | **Lower limit (95%)** | **Upper limit (95%)** |
|---|---|---|---|
| **Sensitivity** | 95.7 | 78.1 | 99.9 |
| **Specificity** | 92.0 | 74.0 | 99.0 |

**Table 4: Sensitivity and specificity related to the ratio A₃/A₁**

| **A₃/A₁** | **Estimate (%)** | **Lower limit (95%)** | **Upper limit (95%)** |
|---|---|---|---|
| **Sensitivity** | 95.7 | 78.1 | 99.9 |
| **Specificity** | 96.0 | 79.6 | 99.9 |

Table 5 shows the AUC values calculated from the ROC curves identified in Figures 6a and 6b, as well as the lower limit and the upper limit, calculated taking into account a 95% confidence interval. In both cases the calculated AUC value is greater than 0.9 and this, in fact, makes the test highly accurate.

**Table 5: AUC values obtained and relative limits**

| **Areas Ratio** | **AUC** | **Lower limit (95%)** | **Upper limit (95%)** |
|---|---|---|---|
| **A₂/A₁** | 0.97739 | 0.94436 | 1.0 |
| **A₃/A₁** | 0.98087 | 0.94783 | 1.0 |

Phenylalanine was selected as the first indicator; phospholipids were selected as the second indicator; and amide-I was selected as the third indicator.

The first band is within a first sub-range of wavenumbers, preferably between about 1015 cm⁻¹ and 990 cm⁻¹; the second band is within a second sub-range of wavenumbers, preferably between about 1500 cm⁻¹ and 1400 cm⁻¹; and the third band is within a third sub-range of wavenumbers, preferably between about 1750 cm⁻¹ and 1550 cm⁻¹.

In particular, the third band, for example centered near the 1650 cm⁻¹, was assigned to the vibration modes of the amide-I, which mainly involves *C=O* stretching and, to a lesser extent, *C-N* stretching, *C_{α}-C-N* bending vibrations, and *N-H* bending vibrations in plane of the peptide groups.

The second band, for example centered near the 1450 cm⁻¹, was assigned to the phospholipid vibration modes which involve the bending vibrations of the groups *CH₂* and *CH₃*; while the first band, for example, centered near the 1005 cm⁻¹, was assigned to the phenylalanine vibration mode, which involves the breathing of the phenylalanine aromatic ring.

Preferably, the first threshold value and the second threshold value can be defined by performing the following steps:
- providing a Raman spectrum of a blood serum sample for each patient of a first known group of celiac patients and for each patient of a second known group of non-celiac patients;
- for each patient, both of the first group and of the second group, selecting from the respective Raman spectrum the first band characteristic of the first indicator of the presence of celiac disease, the second band characteristic of the second indicator of the presence of celiac disease, and the third band characteristic of the third indicator of the presence of celiac disease;
- for each Raman spectrum, calculating the first ratio A₂/A₁ and the second ratio A₃/A₁, where A₁ is the area under the first band, A₂ is the area under the second band, and A₃ is the area under the third band;
- performing an analysis of a first ROC curve, obtained by considering the first ratios A₂/A₁ as a database, and determining, in a known manner, the first optimal threshold value using the Youden index, where the aforesaid Youden index is obtained by means of the homonymous function, which depends on sensitivity and specificity, which in turn depend on the considered cut-off value. The cut-off value at which the Youden index is maximum therefore represents the optimal cut-off value;
- performing an analysis of a second ROC curve, obtained by considering the second ratios A₃/A₁ as the database, and determining, similarly to what was described above, the second optimal threshold value using the Youden index.

As is known, most diagnostic tests produce a quantitative result. To discriminate between healthy and ill people it is necessary to have a threshold or cut-off value. In an ideal situation, healthy and ill people return different test values and the cut-off value is immediately determined. In real situations, there is always some overlap in the distribution of healthy and ill people. Sensitivity and specificity are inversely related in relation to the selection of the cut-off. The adoption of a threshold which offers high sensitivity leads to a loss of specificity and vice versa.

In the proposed method, although the distribution of healthy and ill people is distinct enough, to obtain the threshold values which minimize the probability of finding false positives and false negatives, it is preferable to construct two ROC (Receiver Operating Characteristic) curves in a known manner, with sensitivity on the ordinates and (1-specificity) on the abscissas, considering the first ratios A₂/A₁ and the second ratios A₃/A₁, respectively, as the database, therefore obtaining the first optimal threshold value and the second optimal threshold value.

It was found that the second threshold value, related to the A₃/A₁ ratios, is greater than the first threshold value, related to the A₂/A₁ ratios.

## Claims

1. A diagnosis method for detecting celiac disease comprising the following steps:
a) providing as input data a Raman spectrum of a blood serum sample;
b) selecting from the Raman spectrum a first band characteristic of a first indicator of presence of celiac disease, a second band characteristic of a second indicator of presence of celiac disease, and a third band characteristic of a third indicator of presence of celiac disease;
c) calculating a first ratio A_{2'}/A₁ and a second ratio A_{3'}/A₁, wherein A₁ is the area under the first band, A_{2'} is the area under the second band, and A_{3'} is the area under the third band;
d) verifying that the first ratio A_{2'}/A₁ is greater than a first threshold value and that the second ratio A_{3'}/A₁ is greater than a second threshold value to confirm that the blood serum sample belongs to a celiac patient;
wherein said first indicator is given by phenylalanine; wherein said second indicator is given by phospholipids; and wherein said third indicator is given by amide-I.

2. A method according to claim 1, wherein after the step b) there are provided the steps of
- performing a deconvolution of at least the second band and the third band of the Raman spectrum, obtaining a respective plurality of Gaussians;
- calculating a sum A₂ of the areas of the individual Gaussians related to the second band, and a sum A₃ of the areas of the individual Gaussians related to the third band;
- calculating a ratio A₂/A₁ and a ratio A₃/A₁;
- verifying that the ratio A₂/A₁ is greater than the first threshold value and that the ratio A₃/A₁ is greater than the second threshold value to confirm that the blood serum sample belongs to a celiac patient.

3. A method according to claim 1 or 2, wherein said first indicator is given by the breathing vibration mode of the phenylalanine aromatic ring; wherein said second indicator is given by the phospholipid vibration modes; and wherein said third indicator is given by the amide-I vibration modes.

4. A method according to any one of the preceding claims, wherein the first band is comprised in a first sub-range of wavenumbers, the second band is comprised in a second sub-range of wavenumbers, and the third band is comprised in a third sub-range of wavenumbers.

5. A method according to claim 4, wherein said first sub-range of wavenumbers is between about 1015 cm⁻¹ and 990 cm⁻¹, said second sub-range of wavenumbers is between about 1500 cm⁻¹ and 1400 cm⁻¹, and said third sub-range of wavenumbers is between about 1750 cm⁻¹ and 1550 cm⁻¹.

6. A method according to any one of claims from 2 to 5, wherein said first threshold value and said second threshold value can be defined by performing the following steps:
- providing a Raman spectrum of a blood serum sample for each patient of a first known group of celiac patients and for each patient of a second known group of non-celiac patients;
- for each patient, both of the first group and of the second group, selecting from the respective Raman spectrum the first band characteristic of the first indicator of the presence of celiac disease, the second band characteristic of the second indicator of the presence of celiac disease, and the third band characteristic of the third indicator of the presence of celiac disease;
- performing for each Raman spectrum a deconvolution of at least the second band and the third band, obtaining a respective plurality of Gaussians; calculating the sum A₂ of the areas of the individual Gaussians related to the second band, and the sum A₃ of the areas of the individual Gaussians related to the third band; and calculating the ratio A₂/A₁ and the ratio A₃/A₁, wherein A₁ is the area under the first band of the Raman spectrum;
- performing an analysis of a first ROC curve considering as database the ratios A₂/A₁ and calculating the first threshold value by means of the Youden's index; and performing an analysis of a second ROC curve considering as database the ratios A₃/A₁ and calculating the second threshold value by means of Youden's index.

7. A method according to any one of the preceding claims, wherein said second threshold value is greater than said first threshold value.

8. A method according to any one of the preceding claims, wherein the Raman spectrum is acquired in a range of wavenumbers between 3500 cm⁻¹ and 300 cm⁻¹.

9. A method according to any one of the preceding claims, wherein, in order to obtain the Raman spectrum of the blood serum sample, a Raman spectrometer is used.

10. A method according to claim 9, wherein a detector is connected to said Raman spectrometer.

11. A method according to claim 9 or 10, wherein the Raman spectrum is acquired by performing a number of scans of the serum sample between 25 and 35, with an exposure time between 30 and 90 seconds for each scan.

12. A method according to any one of the preceding claims, wherein in step c) the parameter selected to perform the deconvolution, in addition to the respective band of the Raman spectrum, is the full width at half maximum (FWHM) of the respective band, expressed in wavenumbers.

13. A method according to claim 8, wherein the Raman spectrum is acquired in a range of wavenumbers between 3300 cm⁻¹ and 400 cm⁻¹.

14. A method according to claim 13, wherein the Raman spectrum is acquired in a range of wavenumbers between 2500 cm⁻¹ and 500 cm⁻¹.

15. A method according to claim 10, wherein said detector is a charge-coupled device (CCD).

16. A method according to claim 15, wherein said detector is integrated with a monochromator.

17. A method according to any one of claims from 2 to 16, wherein between the step of performing a deconvolution of at least the second band and the third band of the Raman spectrum, obtaining a respective plurality of Gaussians, and the step of calculating the sum A₂ of the areas of the individual Gaussians related to the second band, and the sum A₃ of the areas of the individual Gaussians related to the third band,
a cleaning of the individual Gaussians related to the second band and third band of the spectrum from any possible observed noise signal is provided.

## Patentansprüche

1. Diagnoseverfahren zum Detektieren von Zöliakie, umfassend die folgenden Schritte:
a) Bereitstellen eines Raman-Spektrums einer Blutserumprobe als Eingangsdaten;
b) Auswählen eines ersten Bandes, das für einen ersten Indikator für das Vorhandensein von Zöliakie charakteristisch ist, eines zweiten Bandes, das für einen zweiten Indikator für das Vorhandensein von Zöliakie charakteristisch ist, und eines dritten Bandes, das für einen dritten Indikator für das Vorhandensein von Zöliakie charakteristisch ist, aus dem Raman-Spektrum;
c) Berechnen eines ersten Verhältnisses A_{2'}/A₁ und eines zweiten Verhältnisses A₃/A₁, wobei A₁ die Fläche unter dem ersten Band ist, A_{2'} die Fläche unter dem zweiten Band ist und A_{3'} die Fläche unter dem dritten Band ist;
d) Verifizieren, dass das erste Verhältnis A_{2'}/A₁ größer als ein erster Schwellenwert ist und dass das zweite Verhältnis A_{3'}/A₁ größer als ein zweiter Schwellenwert ist, um zu bestätigen, dass die Blutserumprobe zu einem Zöliakiepatienten gehört;
wobei der erste Indikator durch Phenylalanin gegeben ist;
wobei der zweite Indikator durch Phospholipide gegeben ist; und wobei der dritte Indikator durch Amid-I gegeben ist.

2. Verfahren nach Anspruch 1, wobei nach dem Schritt b) die folgenden Schritte vorgesehen sind:
- Durchführen einer Entfaltung von mindestens dem zweiten Band und dem dritten Band des Raman-Spektrums, um eine jeweilige Vielzahl von Gaußschen zu erhalten;
- Berechnen einer Summe A₂ der Flächen der einzelnen Gaußschen, die sich auf das zweite Band beziehen, und einer Summe A₃ der Flächen der einzelnen Gaußschen, die sich auf das dritte Band beziehen;
- Berechnen eines Verhältnisses A₂/A₁ und eines Verhältnisses A₃/A₁;
- Verifizieren, dass das Verhältnis A₂/A₁ größer als der erste Schwellenwert ist und dass das Verhältnis A₃/A₁ größer als der zweite Schwellenwert ist, um zu bestätigen, dass die Blutserumprobe zu einem Zöliakiepatienten gehört.

3. Verfahren nach Anspruch 1 oder 2, wobei der erste Indikator durch den Atemschwingungsmodus des aromatischen Phenylalaninrings gegeben ist; wobei der zweite Indikator durch die Phospholipidschwingungsmodi gegeben ist; und wobei der dritte Indikator durch die Amid-I-Schwingungsmodi gegeben ist.

4. Verfahren nach einem der vorhergehenden Ansprüche, wobei das erste Band in einem ersten Teilbereich von Wellenzahlen enthalten ist, das zweite Band in einem zweiten Teilbereich von Wellenzahlen enthalten ist und das dritte Band in einem dritten Teilbereich von Wellenzahlen enthalten ist.

5. Verfahren nach Anspruch 4, wobei der erste Teilbereich von Wellenzahlen zwischen etwa 1015 cm⁻¹ und 990 cm⁻¹ liegt, der zweite Teilbereich von Wellenzahlen zwischen etwa 1500 cm⁻¹ und 1400 cm⁻¹ liegt und der dritte Teilbereich von Wellenzahlen zwischen etwa 1750 cm⁻¹ und 1550 cm⁻¹ liegt.

6. Verfahren nach einem der Ansprüche 2 bis 5, wobei der erste Schwellenwert und der zweite Schwellenwert durch Durchführen der folgenden Schritte definiert werden können:
- Bereitstellen eines Raman-Spektrums von einer Blutserumprobe für jeden Patienten von einer ersten
bekannten Gruppe von Zöliakiepatienten und für jeden Patienten von einer zweiten bekannten Gruppe von
Nicht-Zöliakiepatienten;
- Auswählen, für jeden Patienten, sowohl der ersten Gruppe als auch der zweiten Gruppe, aus dem jeweiligen Raman-Spektrum, des ersten Bandes, das für den ersten Indikator für das Vorhandensein von Zöliakie charakteristisch ist, des zweiten Bandes, das für den zweiten Indikator für das Vorhandensein von Zöliakie charakteristisch ist, und des dritten Bandes, das für den dritten Indikator für das Vorhandensein von Zöliakie charakteristisch ist;
- Durchführen für jedes Raman-Spektrum einer Entfaltung von mindestens dem zweiten Band und dem dritten Band, Erhalten einer jeweiligen Vielzahl von Gaußschen; Berechnen der Summe A₂ der Flächen der einzelnen Gaußschen, die sich auf das zweite Band beziehen, und der Summe A₃ der Flächen der einzelnen Gaußschen, die sich auf das dritte Band beziehen; und Berechnen des Verhältnisses A₂/A₁ und des Verhältnisses A₃/A₁, wobei A₁ die Fläche unter dem ersten Band des Raman-Spektrums ist;
- Durchführen einer Analyse einer ersten ROC-Kurve unter Berücksichtigung der Verhältnisse A₂/A₁ als Datenbank und Berechnen des ersten Schwellenwerts mittels des Youden-Index; und Durchführen einer Analyse einer zweiten ROC-Kurve unter Berücksichtigung der Verhältnisse A₃/A₁ als Datenbank und Berechnen des zweiten Schwellenwerts mittels des Youden-Index.

7. Verfahren nach einem der vorhergehenden Ansprüche, wobei der zweite Schwellenwert größer als der erste Schwellenwert ist.

8. Verfahren nach einem der vorhergehenden Ansprüche, wobei das Raman-Spektrum in einem Bereich von Wellenzahlen zwischen 3500 cm⁻¹ und 300 cm⁻¹ erfasst wird.

9. Verfahren nach einem der vorhergehenden Ansprüche, wobei zum Erhalten des Raman-Spektrums der Blutserumprobe ein Raman-Spektrometer verwendet wird.

10. Verfahren nach Anspruch 9, wobei ein Detektor mit dem Raman-Spektrometer verbunden ist.

11. Verfahren nach Anspruch 9 oder 10, wobei das Raman-Spektrum durch Durchführen einer Anzahl von Scans der Serumprobe zwischen 25 und 35 mit einer Belichtungszeit zwischen 30 und 90 Sekunden für jeden Scan erfasst wird.

12. Verfahren nach einem der vorhergehenden Ansprüche, wobei in Schritt c) der zur Durchführung der Entfaltung ausgewählte Parameter zusätzlich zu dem jeweiligen Band des Raman-Spektrums die Halbwertsbreite (FWHM) des jeweiligen
Bandes, ausgedrückt in Wellenzahlen, ist.

13. Verfahren nach Anspruch 8, wobei das Raman-Spektrum in einem Bereich von Wellenzahlen zwischen 3300 cm⁻¹ und 400 cm⁻¹ erfasst wird.

14. Verfahren nach Anspruch 13, wobei das Raman-Spektrum in einem Bereich von Wellenzahlen zwischen 2500 cm⁻¹ und 500 cm⁻¹ erfasst wird.

15. Verfahren nach Anspruch 10, wobei der Detektor eine ladungsgekoppelte Vorrichtung (charge-coupled device - CCD) ist.

16. Verfahren nach Anspruch 15, wobei der Detektor mit einem Monochromator integriert ist.

17. Verfahren nach einem der Ansprüche 2 bis 16, wobei zwischen dem Schritt des Durchführens einer Entfaltung von mindestens dem zweiten Band und dem dritten Band des Raman-Spektrums, des Erhaltens einer jeweiligen Vielzahl von Gaußschen, und dem Schritt des Berechnens der Summe A₂ der Flächen der einzelnen Gaußschen, die sich auf das zweite Band beziehen, und der Summe A₃ der Flächen der einzelnen Gaußschen, die sich auf das dritte Band beziehen, eine Reinigung der einzelnen Gaußschen, die sich auf das zweite Band und das dritte Band des Spektrums beziehen, von einem möglichen beobachteten Rauschsignal bereitgestellt wird.

## Revendications

1. Méthode de diagnostic de maladie coeliaque comprenant les étapes suivantes :
a) fournir comme données d'entrée un spectre Raman d'un échantillon de sérum sanguin ;
b) sélectionner dans le spectre Raman une première bande caractéristique d'un premier indicateur de la présence de la maladie coeliaque, une deuxième bande caractéristique d'un deuxième indicateur de la présence de la maladie coeliaque et une troisième bande caractéristique d'un troisième indicateur de la présence de la maladie coeliaque ;
c) calculer un premier rapport A_{2'}/A1 et un second rapport A_{3'}/A₁, dans lequel A₁ est la surface sous la première bande, A_{2'} est la surface sous la deuxième bande, et A_{3'} est la surface sous la troisième bande ;
d) vérifier que le premier rapport A_{2'}/A₁ est supérieur à une première valeur seuil et que le second rapport A_{3'}/A₁ est supérieur à une seconde valeur seuil pour confirmer que l'échantillon de sérum sanguin appartient à un patient coeliaque ;
dans laquelle ledit premier indicateur est donné par la phénylalanine ;
dans laquelle ledit deuxième indicateur est donné par les phospholipides ; et dans lequel ledit troisième indicateur est donné par l'amide-I.

2. Méthode selon la revendication 1, dans laquelle l'étape b) est suivie des étapes suivantes
- effectuer une déconvolution d'au moins la deuxième bande et la troisième bande du spectre Raman, en obtenant une pluralité respective de gaussiennes ;
- calculer une somme A₂ des aires des gaussiennes individuelles liées à la deuxième bande, et une somme A₃ des aires des gaussiennes individuelles liées à la troisième bande ;
- le calcul d'un rapport A₂/A₁ et d'un rapport A₃/A₁;
- vérifier que le rapport A₂/A₁ est supérieur à la première valeur seuil et que le rapport A₃/A₁ est supérieur à la seconde valeur seuil pour confirmer que l'échantillon de sérum sanguin appartient à un patient coeliaque.

3. Méthode selon la revendication 1 ou 2, dans laquelle ledit premier indicateur est donné par le mode de vibration respiratoire du cycle aromatique de la phénylalanine ; dans laquelle ledit deuxième indicateur est donné par les modes de vibration du phospholipide ; et dans lequel ledit troisième indicateur est donné par les modes de vibration de l'amide-I.

4. Méthode selon l'une quelconque des revendications précédentes, dans laquelle la première bande est comprise dans une première sous-gamme de nombres d'ondes, la deuxième bande est comprise dans une deuxième sous-gamme de nombres d'ondes, et la troisième bande est comprise dans une troisième sous-gamme de nombres d'ondes.

5. Méthode selon la revendication 4, dans laquelle ladite première sous-gamme de nombres d'ondes est comprise entre environ 1015 cm⁻¹ et 990 cm⁻¹, ladite deuxième sous-gamme de nombres d'ondes est comprise entre environ 1500 cm⁻¹ et 1400 cm⁻¹, et ladite troisième sous-gamme de nombres d'ondes est comprise entre environ 1750 cm⁻¹ et 1550 cm⁻¹.

6. Procédé selon l'une quelconque des revendications de 2 à 5, dans lequel ladite première valeur seuil et ladite seconde valeur seuil peuvent être définies en effectuant les étapes suivantes :
- fournir un spectre Raman d'un échantillon de sérum sanguin pour chaque patient d'un premier
groupe connu de patients coeliaques et pour chaque patient d'un second groupe connu de
patients non coeliaques ;
- pour chaque patient, à la fois du premier groupe et du second groupe, en sélectionnant dans le spectre Raman respectif la première bande caractéristique du premier indicateur de la présence de la maladie coeliaque, la deuxième bande caractéristique du deuxième indicateur de la présence de la maladie coeliaque, et la troisième bande caractéristique du troisième indicateur de la présence de la maladie coeliaque ;
- effectuer pour chaque spectre Raman une déconvolution au moins de la deuxième bande et de la troisième bande, en obtenant une pluralité respective de gaussiennes ; en calculant la somme A₂ des aires des gaussiennes individuelles liées à la deuxième bande, et la somme A₃ des aires des gaussiennes individuelles liées à la troisième bande ; et en calculant le rapport A₂/A₁ et le rapport A₃/A₁, dans lequel A₁ est l'aire sous la première bande du spectre Raman ;
- effectuer une analyse d'une première courbe ROC en considérant comme base de données les rapports A₂/A₁ et en calculant la première valeur seuil au moyen de l'indice de Youden ; et effectuer une analyse d'une deuxième courbe ROC en considérant comme base de données les rapports A₃/A₁ et en calculant la deuxième valeur seuil au moyen de l'indice de Youden.

7. Méthode selon l'une quelconque des revendications précédentes, dans laquelle ladite seconde valeur seuil est supérieure à ladite première valeur seuil.

8. Méthode selon l'une quelconque des revendications précédentes, dans laquelle le spectre Raman est acquis dans une gamme de nombres d'ondes comprise entre 3500 cm⁻¹ et 300 cm⁻¹.

9. Méthode selon l'une quelconque des revendications précédentes, dans laquelle un spectre Raman est utilisé pour obtenir le spectre Raman de l'échantillon de sérum sanguin.

10. Méthode selon la revendication 9, dans laquelle un détecteur est connecté au spectromètre Raman.

11. Méthode selon la revendication 9 ou 10, dans laquelle le spectre Raman est acquis en effectuant un nombre de balayages de l'échantillon de sérum compris entre 25 et 35, avec un temps d'exposition compris entre 30 et 90 secondes pour chaque balayage.

12. Procédé selon l'une quelconque des revendications précédentes, dans lequel, à l'étape c), le paramètre sélectionné pour effectuer la déconvolution, en plus de la bande respective du spectre Raman, est la largeur totale à mi-maximum (FWHM) de la bande respective,
exprimée en nombres d'ondes.

13. Méthode selon la revendication 8, dans laquelle le spectre Raman est acquis dans une gamme de nombres d'ondes comprise entre 3300 cm⁻¹ et 400 cm⁻¹.

14. Méthode selon la revendication 13, dans laquelle le spectre Raman est acquis dans une gamme de nombres d'ondes comprise entre 2500 cm⁻¹ et 500 cm⁻¹.

15. Méthode selon la revendication 10, dans laquelle ledit détecteur est un dispositif à couplage de charge (CCD).

16. Méthode selon la revendication 15, dans laquelle ledit détecteur est intégré à un
monochromateur.

17. Procédé selon l'une quelconque des revendications 2 à 16, dans lequel entre l'étape consistant à effectuer une déconvolution d'au moins la deuxième bande et la troisième bande du spectre Raman, en obtenant une pluralité respective de gaussiennes, et l'étape consistant à calculer la somme A₂ des aires des gaussiennes individuelles liées à la deuxième bande, et la somme A₃ des aires des gaussiennes individuelles liées à la troisième bande,
un nettoyage des gaussiennes individuelles liées à la deuxième bande et à la troisième bande du spectre à partir de tout signal de bruit éventuellement observé est assuré.
